# EUROPEAN PATENT APPLICATION

(11) **EP 4 414 104 A1**
(43) Date of publication of application: **14.08.2024**
(21) Application number: 23155414.8
(22) Date of filing: 07.02.2023
(51) Int. Cl.: B22F 5/10, B22F 10/28, B22F 10/38, B22F 12/41, B33Y 80/00, C22C 1/04

(54) **ADDITIVE MANUFACTURING OF A MICROSTRUCTURE**

(71) Applicant: Koninklijke Philips N.V., 5656 AG Eindhoven (NL)
(72) Inventor: UR RAHMAN, Naveed, Eindhoven (NL); JORRITSMA, Jorrit, Eindhoven (NL); HAFKAMP, Thomas, 5656AG Eindhoven (NL); GRAGE, Jan, Eindhoven (NL); DE KLOET, Ron, Eindhoven (NL)
(74) Representative: Philips Intellectual Property & Standards

(57) **Abstract**

The invention relates to a method for additive manufacturing of a microstructure (10) for selective transmission of X-ray or gamma-ray radiation. The method includes depositing (110) a layer of powder on a build plate or on a layer from at least one previous manufacturing step on the build plate, wherein the layer of powder comprises a high-Z material powder. Furthermore, the method includes selectively melting (120) and solidifying the powder to form septa wall structures (20) by scanning an electron beam in a spot sequence on the layer of powder to sequentially expose a plurality of spots (40) on the layer of powder to an electron beam spot. A size of the electron beam spot is less than 150 µm in diameter, preferably less than 75 µm in diameter, and more preferably equal to or less than 50 µm in diameter.

## Description

### FIELD OF THE INVENTION

The invention relates to a method for additive manufacturing, more specifically to a method for additive manufacturing of a microstructure for selective transmission of X-ray or gamma-ray radiation.

### BACKGROUND OF THE INVENTION

Additive manufacturing technologies such as direct metal laser sintering, or laser powder bed fusion, show great promise for manufacturing of X-ray and gamma ray transmission selective microstructures like anti-scatter devices, 2D anti-scatter grids etc. In some cases, such as for microstructures with high density, high purity and/or low micro-cracking, other manufacturing technologies may be needed.

Electron beam powder bed fusion (E-PBF) uses an electron beam to selectively melt a metal powder into solid material in a vacuum chamber. E-PBF can result in lower residual material stresses and reduced cracks thanks to higher build temperatures. E-PBF is commonly used to build microstructures with materials such as titanium-aluminum, cobalt-chrome, or nickel-based alloys.

Research Disclosure #697065 suggests feasibility to additively manufacture metal parts in refractory metals such as tungsten and alloys thereof, using E-PBF.

Known E-PBF approaches in the literature are not suitable for manufacturing of X-ray and gamma ray transmission selective microstructures, such as anti-scatter grids, since this kind of transmission selective microstructures may advantageously have high surface quality, high resolution, high septa wall density etc. Hence, there is a need to improve the manufacturing of these microstructures.

### SUMMARY OF THE INVENTION

It is an object of the invention to provide improved manufacturing of microstructures for selective transmission of X-ray or gamma-ray radiation.

The invention is defined by the independent claims. Advantageous embodiments are defined in the dependent claims.

According to a first aspect of the invention, there is provided a method for additive manufacturing of a microstructure for selective transmission of X-ray or gamma-ray radiation. The method comprises:
depositing a layer of powder on a build plate or on a layer from at least one previous manufacturing step on the build plate, wherein the layer of powder comprises a high-Z material powder; and
selectively melting and solidifying the powder to form septa wall structures by scanning an electron beam in a spot sequence on the layer of powder to sequentially expose a plurality of spots on the layer of powder to an electron beam spot, wherein a size of the electron beam spot is less than 150 µm in diameter, preferably less than 75 µm in diameter, and more preferably equal to or less than 50 µm in diameter.

The proposed manufacturing method enables high productivity of microstructures with high density septa walls for selective transmission of X-ray or gamma-ray radiation. The structures may be built without support structures and have little or no micro-cracking and excellent surface properties compared to previously known manufacturing technologies. Generally, it is known that a high electron beam density for powder bed spot melting may cause melting issues such as charging and smoke events. However, the inventors surprisingly found that in combination with a high-Z material powder it is advantageous to use a very small electron beam spot size, and thus, for a given electron beam power, a very high beam energy density for spot melting of the septa wall structures. Thanks to the very high material density of a high Z material, in combination with the small electron beam spot size, the method enables high resolution manufacturing of the microstructures. The current of the electron beam may be e.g. but not limited to 10-100 mA and the scanning speed e.g. up to or even larger than 2 m/s.

A high-Z material is a material (element) with a high atomic number Z. The high-Z material is preferably a metal. The high-Z material may preferably be Tungsten (Wolfram, W, with atomic number 74). Other high-Z materials, such as but not limited to, Molybdenum (Mo, atomic number 42), Tantalum (Ta, atomic number 73), Niobium (Nb, atomic number 41), Lead (Pb, atomic number 82), Bismuth (Bi, atomic number 83), Rhenium (Re, atomic number 75), Silver (Ag, atomic number 47) or Gold (Au, atomic number 79) may alternatively or additionally or in combination be used. So, in the context of this disclosure, a high-Z material preferably has an atomic number Z exceeding 40, and more preferably exceeding 70. The high-Z material powder may alternatively or additionally or in combination comprise an alloy including at least one high-Z material.

According to an embodiment of the invention, the high-Z material powder has a particle size distribution with a median diameter of less than 50 µm, preferably with a median diameter of less than 25 µm, most preferably less than or equal to 20 µm. Despite the very high energy density of the beam, it was found that optimal results may be achieved with a powder having a small particle size distribution.

According to an embodiment of the invention, the deposited layer of powder has a layer thickness between 10 µm and 50 µm. Thin layers of the powders, e.g. in combination with small particle size and the narrow electron beam, enables high resolution manufacturing of high density septa walls.

According to an embodiment of the invention, the method further comprises selectively pre-heating spots of the powder by scanning the electron beam in a spot pre-sintering sequence on the layer of powder, and wherein the spot pre-sintering sequence is carried out before the spot sequence. By pre-heating or pre-sintering spots of powder with the electron beam, subsequent melting of the powder with the spot-sequence can be improved.

According to an embodiment of the invention, a scanning direction and sequence of spots of the pre-sintering sequence is the same as a scanning direction and sequence of spots of the spot sequence for all spots, or, a scanning direction and sequence of spots of the pre-sintering sequence is the same as a scanning direction and sequence of spots of the spot sequence for a plurality of spots. Pre-heating and melting the powder according to the same or partly the same pattern, may be advantageous for the formation of septa walls with high density.

According to an embodiment of the invention, the septa wall structures of the microstructure comprise first wall structures formed with each a respective length axis parallel to a first direction and second wall structures formed with each a respective length axis parallel to a second direction, and wherein the second direction is at an angle to the first direction. With septa walls in two different directions at an angle, such as but not limited to e.g. 90 degrees or 60 degrees or 45 degrees, it is possible to advantageously manufacture grid microstructures, such as radiation anti scatter grids.

According to an embodiment of the invention, the spot sequence to sequentially expose a plurality of spots on the layer of powder to the electron beam spot comprises a first sequence followed by a second sequence, wherein the first sequence comprises scanning the electron beam spot to sequentially expose a plurality of spots on the length axes parallel to the first direction, and wherein the second sequence comprises scanning the electron beam spot to sequentially expose a plurality of spots on the length axes parallel to the second direction. This dedicated spot melting strategy with respect to the length axes of the formed septa walls may reduce the amount of sintered powder within a pixel of a grid to enable high resolution. Furthermore, the strategy may be used to achieve a desirable grain structure in each of the septa walls.

According to an embodiment of the invention, the first sequence comprises scanning the electron beam spot along a plurality of first septa wall paths on the respective length axes parallel to the first direction, and wherein the second sequence comprises scanning the electron beam spot along a plurality of second septa wall paths on the respective length axes parallel to the second direction. By scanning the electron beam spot first along septa wall paths corresponding to the first direction, and then along septa wall paths corresponding to the second direction it is possible to achieve advantageous manufacturing of septa walls in the respective directions. Scanning along a septa wall path in this context means that adjacent spots on the path are sequentially exposed to the electron beam spot, which thus moves along the path. It should be noted that the movement of the spot along a first septa wall path may be either parallel or anti-parallel to the first direction. Similarly, movement of the spot along a second septa wall path may be either parallel or anti-parallel to the second direction.

According to a second aspect of the invention, there is provided a microstructure for selective transmission of X-ray or gamma-ray radiation, obtainable by the method according to the first aspect or any embodiment thereof.

According to an embodiment of the invention, the septa wall structures have a wall thickness of less than 200 µm, preferably less than 100 µm, more preferably less than or equal to 70 µm.

According to an embodiment of the invention, the septa wall structures have a height to thickness aspect ratio of more than 100, preferably more than 200 and even more preferably more than 400. Manufacturing of microstructures with thin septa walls, such as but not limited to septa walls with a thickness of less than 100 µm, and particularly septa walls with a very large height to thickness ratio, such as but not limited to thin septa walls with a height of at least 40 mm, may enable microstructures with precise control of transmission of X-ray or gamma-ray radiation. Such as, but not limited to, microstructures that efficiently reduce scattering of radiation, while having low losses in radiation intensity due to thickness of the walls.

According to a further aspect of the invention, there is provided an imaging component comprising the microstructure.

According to an embodiment of the invention, the imaging component comprises a plurality of stacked microstructures. Stacking of multiple microstructures to form the imaging component may be advantageous for improved simplicity of manufacturing.

According to an embodiment of the invention, the imaging component comprises one or more of:
- an X-ray or gamma-ray filter;
- an X-ray or gamma-ray collimator;
- an X-ray or gamma-ray anti-scatter device; and
- an X-ray or gamma-ray grating.

These and other aspects of the invention will be apparent from and elucidated with reference to the embodiments described hereinafter.

### BRIEF DESCRIPTION OF THE DRAWINGS

Fig. 1 schematically illustrates a method for additive manufacturing of a microstructure for selective transmission of X-ray or gamma-ray radiation, according to an embodiment of the invention.
Fig. 2 schematically illustrates a method for additive manufacturing of a microstructure for selective transmission of X-ray or gamma-ray radiation, where the method includes a selective pre-heating step, according to an embodiment of the invention.
Fig. 3a and Fig. 3b schematically illustrate a microstructure for selective transmission of X-ray or gamma-ray radiation, according to an embodiment of the invention.
Fig. 4a and Fig. 4b schematically illustrate spot sequences for additive manufacturing of a microstructure for selective transmission of X-ray or gamma-ray radiation, according to an embodiment of the invention.
Fig. 5 shows optical surface images of a septa wall produced by a method of additive manufacturing according to an embodiment of the invention.
Fig. 6 shows an image of individual septa walls produced by a method of additive manufacturing according to an embodiment of the invention.

### DETAILD DESCRIPTION OF EMBODIMENTS

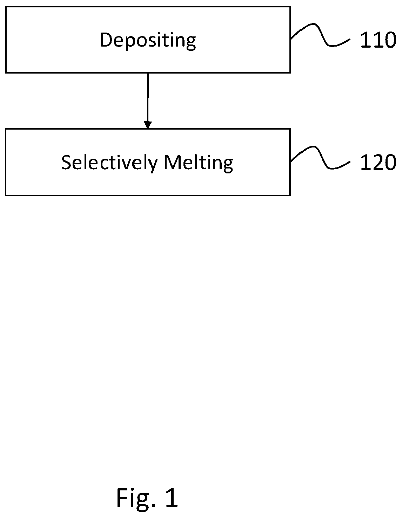

A method for additive manufacturing of a microstructure 10 for selective transmission of X-ray or gamma-ray radiation, according to an embodiment of the invention, is illustrated with a flowchart in Fig. 1. The method in Fig. 1 includes a step of depositing 110 a layer of powder on a build plate or on a layer from at least one previous manufacturing step on the build plate, wherein the layer of powder comprises a high-Z material powder. The high-Z material may include e.g. Tungsten, Molybdenum, Tantalum, Niobium, Lead, Bismuth, Rhenium, Silver or Gold. Combinations of high-Z materials and/or alloys comprising at least one high-Z material may be used. As a non-limiting example, a high purity high-Z material powder may be used in combination with the method. In this way, it may be possible to achieve very high purity septa walls. It may be particularly advantageous to use a high-Z material powder with a purity (in weight % of high-Z material) above 99%, preferably above 99.9%. Such as e.g. a high purity Tungsten powder with a purity above 99%, preferably above 99.9%. The high-Z material powder may have a particle size distribution with a median diameter of less than 50 µm, preferably with a median diameter of less than 25 µm, most preferably less than or equal to 20 µm. Despite a very high energy density of the beam, such as but not limited to an electron beam with a beam current up to or even larger than 100 mA, it was found that optimal results may be achieved with a powder having a small particle size distribution. The deposited layer of powder may advantageously have a layer thickness between 10 µm and 50 µm.

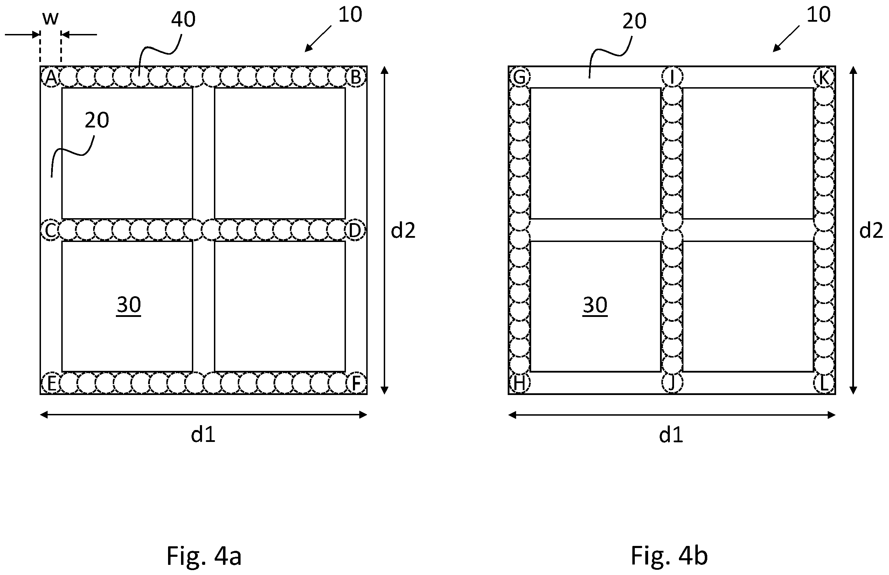

In a next step 120 of the method shown in Fig. 1, the layer of powder is selectively melted and solidified to form septa wall structures 20. This is achieved by scanning an electron beam in a spot sequence on the layer of powder to sequentially expose a plurality of spots 40, such as e.g. shown in Fig. 4a, on the layer of powder to an electron beam spot. For the best results, a size of the electron beam spot may preferably be less than 150 µm in diameter, preferably less than 75 µm in diameter, and more preferably equal to or less than 50 µm in diameter. The method may enable high productivity of microstructures 10 with high density septa walls 20 for selective transmission of X-ray or gamma-ray radiation. The structures may be built without support structures and have little or no micro-cracking and excellent surface properties compared to previously known manufacturing technologies. Thanks to the very high material density (high Z material), in combination with the small electron beam spot size, the method enables high resolution manufacturing of the microstructures. Thin layers of the powder, e.g. in combination with small particle size, may also enable high resolution manufacturing of high density septa walls 20.

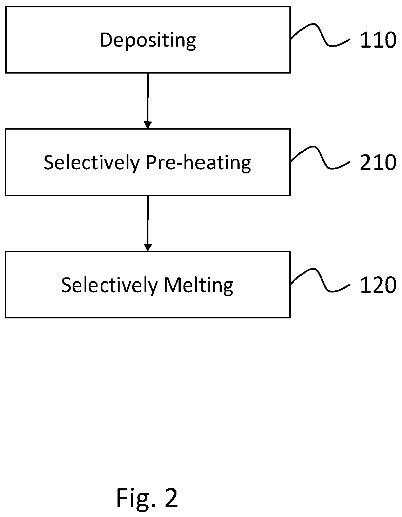

Fig. 2 illustrates a method similar to the method in Fig. 1, with an additional step 210 of selectively pre-heating spots of the powder before the powder is selectively melted and solidified 120 in a spot sequence. In this example, spots 40 of the powder are selectively pre-heated 210 by scanning the electron beam in a spot pre-sintering sequence on the layer of powder. The spot pre-sintering sequence is thus carried out before the spot sequence. By selectively and accurately pre-heating or pre-sintering spots 40 of powder with the electron beam, subsequent melting of the powder with the spot-sequence can be improved. The scanning direction and sequence of spots of the pre-sintering sequence may be the same as a scanning direction and sequence of spots of the spot sequence for all spots 40 or for a plurality of spots 40.

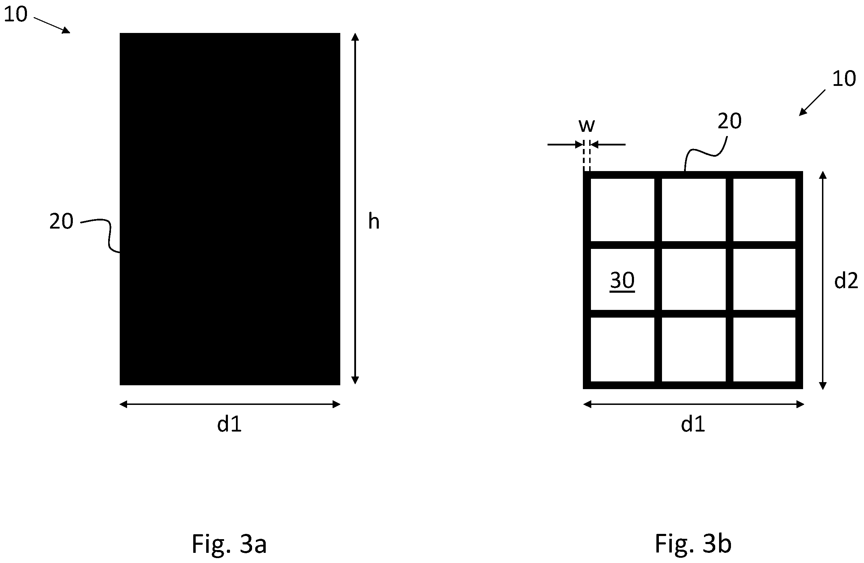

Fig. 3a and Fig. 3b schematically show a microstructure 10 manufactured with the method according to embodiments of the present invention. The microstructure 10 enables selective transmission of X-ray or gamma-ray radiation and may therefore advantageously be used in an imaging component, such as an X-ray or gamma-ray filter, an X-ray or gamma-ray collimator, an X-ray or gamma-ray anti-scatter device, and/or an X-ray or gamma-ray grating. The microstructure 10 may comprise septa wall structures 20 in one or in multiple directions. In the example in Fig. 3a and Fig. 3b, the microstructure 10 is a grid-like structure with septa walls 20 in a first direction d1 and a second direction d2. Such a structure may e.g. be used for two-dimensional anti-scatter grids. In the example in Fig. 3a and Fig. 3b, the angle between septa walls 20 in the first direction d1 and second direction d2 is about 90 degrees. However, other structures with different angles between the walls, such as but not limited to honeycomb like structures, are also conceivable. Fig. 3a shows a side view of the example microstructure 10. In this case the septa wall 20 seen from the side-view of Fig. 3a has a length in first direction d1 and a height h. Fig. 3b illustrates the same structure 10 from above. As seen from the figure, the microstructure 10 has septa walls 20 in the direction of d1 and, in this case orthogonally, in the direction of d2.

The walls have a thickness w. In Fig. 3b, all septa walls have the same thickness w, but it is also possible that different walls have different thickness. The septa walls 20 are dense structures, that may be able to efficiently absorb or reflect radiation. The space between the walls forms transmissive sections 30, which lets radiation pass. With the manufacturing method according to embodiments of the present invention, it is possible to manufacture dense and thin septa walls 20. The septa walls may have a wall thickness w of less than 200 µm, preferably less than 100 µm, or more preferably less than or equal to 70 µm. Each septa wall 20 has a height h and a thickness w. The septa walls 20 may have a height h to thickness w aspect ratio of more than 100, preferably more than 200 and even more preferably more than 400. For example, a septa wall 20 for an anti-scatter grid may advantageously have a thickness w of 70-100 µm and a height h of 40-50 mm. Such microstructures 10 with a large height h to thickness w ratio may enable precise control of transmission of X-ray or gamma-ray radiation through the transmissive sections 30 while having low losses in radiation intensity due to thickness w of the walls 20. For production of imaging components with such microstructures 10, multiple microstructures 10 may be stacked on top of each other and/or tiled together in order to achieve components with desired specification of total height, area etc.

Fig. 4a and Fig. 4b schematically illustrate spot sequences for additive manufacturing of a microstructure for selective transmission of X-ray or gamma-ray radiation, according to an embodiment of the invention. In each spot sequence a plurality of spots 40 on the layer of high-Z material powder are sequentially exposed to an electron beam spot to melt and solidify the powder during the process to form septa wall structures 20. Fig. 4a illustrates spot sequences in the direction d1 and Fig. 4b illustrates spot sequences in the direction d2. The example microstructure 10 in Fig. 4a and Fig. 4b has three septa walls 20 parallel to the direction d1 and three septa walls parallel to the direction d2. In this particular example, d1 is orthogonal to d2 but angles other than 90 degrees between the directions are also possible, depending on the specifications of the microstructure 10 at hand. A total spot sequence to melt (fully or partially) and solidify the powder to build a layer of each of the three-by-three septa walls 20 in Fig. 4a and Fig. 4b may comprise three sequences parallel to the d1 direction and three sequences parallel to the d2 direction. For example, the sequence may begin with three paths along the three septa walls 20 in the d1 direction, such as in Fig. 4a A to B, C to D and E to F, followed by three sequential paths parallel to the direction d2, such as in Fig. 4b G to H, I to J and K to L. For each path, a plurality of spots 40 are sequentially exposed to the electron beam. The electron beam current may be e.g. 10-100 mA and the scanning speed e.g. up to or even larger than 2 m/s. The spot sequence strategy may enable to achieve a desirable grain structure (equiaxed/columnar) of the manufactured septa walls 20. In addition, the amount of sintered powder within a transmissive section (pixel) 30 of the microstructure 10 may be reduced.

Different options of the spot sequence may be considered, such as in Fig. 4a the individual paths may be either from right to left or from left to right figure. Similarly in Fig. 4b the individual paths may be either top to bottom or bottom to top in the figure. For illustration, non-limiting examples of sequences in the d1 direction may be, A to B, D to C and E to F, or F to E, C to D, B to A, or E to F, C to D and A to B, etc. Similarly, different variations top to bottom or bottom to top of the paths in Fig. 4b may be considered. Such dedicated spot melting strategies may reduce the amount of sintered powder within a pixel of a grid to enable high resolution. Furthermore, the strategy may be used to achieve a desirable grain structure in each of the septa walls 20.

In another example, the sequences in Fig. 4a and Fig. 4b may be carried out in an alternating fashion with one sequence in the d1 direction followed by a sequence in the d2 direction. Such as e.g., but not limited to, A to B followed by G to H, C to D followed by I to J, and E to F followed by K to L. Also in this case, many variations of the direction and/or order of sequences are conceivable.

It is noted that the sequences in Fig. 4a and Fig. 4b are only drawn schematically. Any sequence in the d1 or d2 direction may be shorter or longer than what is illustrated in the figures. As a non-limiting example, a sequence in the d1 or d2 direction may be shortened and/or skip a spot 40 such that it does not repeat melting in e.g. spots 40 that are melted in a respective d2 or d1 sequence.

In an example of a method including an additional step 210 of selectively pre-heating spots 40 of the powder before the powder is selectively melted and solidified 120 in a spot sequence, it may be advantageous that a scanning direction and sequence of spots of the pre-sintering sequence is the same or similar as a scanning direction and sequence of spots of the spot sequence. That is, with reference to the example in Fig. 4a and Fig. 4b, to follow the same scanning sequences described above also for selectively pre-heating spots 40 of the powder. This may be the case for all spots 40, or that a scanning direction and sequence of spots of the pre-sintering sequence is the same or similar as a scanning direction and sequence of spots of the spot sequence for a plurality of spots 40. Pre-heating and melting the powder according to the same or partly the same pattern, may be advantageous for the formation of septa walls 20 with high density. Manufacturing in this way to achieve septa walls 20 with a high density may be particularly advantageous for manufacturing of a microstructure 10 for selective transmission of X-ray or gamma-ray radiation, such as structures included in e.g. 2D anti scatter grids.

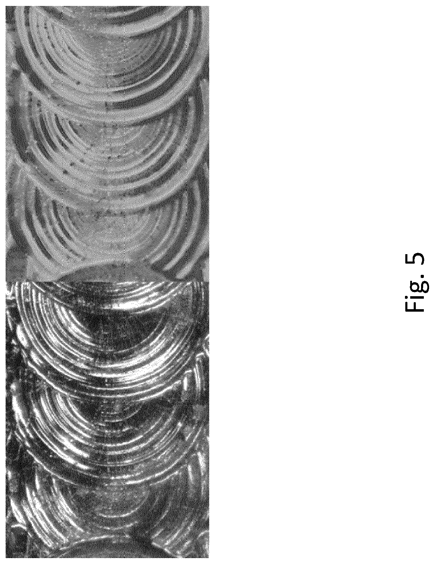

Fig. 5 shows optical surface images of a septa wall produced by a method of additive manufacturing according to an embodiment of the invention. The left and right side of the figure show the same part of the surface under different lighting conditions of an optical microscope. The images clearly illustrate the pattern of individually exposed spots 40 as part of a spot melting strategy.

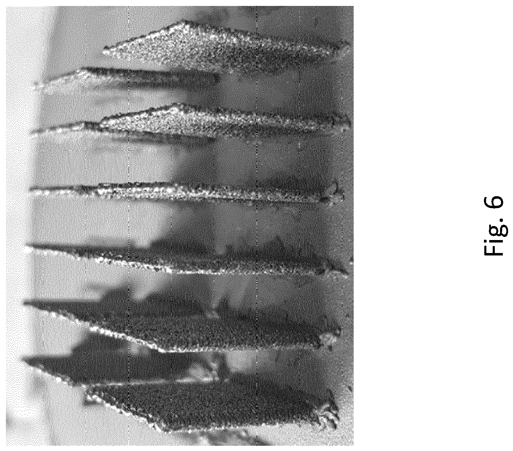

Fig. 6 shows an image of individual septa walls produced by a method of additive manufacturing according to an embodiment of the invention. In this case, multiple walls in a first direction, such as in d1, have been manufactured.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A method for additive manufacturing of a microstructure (10) for selective transmission of X-ray or gamma-ray radiation, the method comprising:
depositing (110) a layer of powder on a build plate or on a layer from at least one previous manufacturing step on the build plate, wherein the layer of powder comprises a high-Z material powder; and
selectively melting (120) and solidifying the powder to form septa wall structures (20) by scanning an electron beam in a spot sequence on the layer of powder to sequentially expose a plurality of spots (40) on the layer of powder to an electron beam spot, wherein a size of the electron beam spot is less than 150 µm in diameter, preferably less than 75 µm in diameter, and more preferably equal to or less than 50 µm in diameter.

2. The method according to claim 1, wherein the high-Z material powder comprises Tungsten, and/or Molybdenum, and/or Tantalum, and/or Niobium, and/or Lead, and/or Bismuth, and/or Rhenium, and/or Silver, and/or Gold.

3. The method according to claim 1 or 2, wherein the high-Z material powder has a particle size distribution with a median diameter of less than 50 µm, preferably with a median diameter of less than 25 µm, most preferably less than or equal to 20 µm.

4. The method according to claim 1 or 2 or 3, wherein the deposited layer of powder has a layer thickness between 10 µm and 50 µm.

5. The method according to any of the preceding claims, wherein the method further comprises selectively pre-heating (210) spots (40) of the powder by scanning the electron beam in a spot pre-sintering sequence on the layer of powder, and wherein the spot pre-sintering sequence is carried out before the spot sequence.

6. The method according to claim 5, wherein a scanning direction and sequence of spots of the pre-sintering sequence is the same as a scanning direction and sequence of spots of the spot sequence for all spots (40), or wherein a scanning direction and sequence of spots of the pre-sintering sequence is the same as a scanning direction and sequence of spots of the spot sequence for a plurality of spots (40).

7. The method according to any of the preceding claims, wherein the septa wall structures (20) of the microstructure (10) comprise first wall structures (20) formed with each a respective length axis parallel to a first direction (d1) and second wall structures (20) formed with each a respective length axis parallel to a second direction (d2), and wherein the second direction (d2) is at an angle to the first direction (d1).

8. The method according to claim 7, wherein the spot sequence to sequentially expose a plurality of spots (40) on the layer of powder to the electron beam spot comprises a first sequence followed by a second sequence, wherein the first sequence comprises scanning the electron beam spot to sequentially expose a plurality of spots (40) on the length axes parallel to the first direction (d1), and wherein the second sequence comprises scanning the electron beam spot to sequentially expose a plurality of spots (40) on the length axes parallel to the second direction (d2).

9. The method according to claim 8, wherein the first sequence comprises scanning the electron beam along a plurality of first septa wall paths on the respective length axes parallel to the first direction (d1), and wherein the second sequence comprises scanning the electron beam along a plurality of second septa wall paths on the respective length axes parallel to the second direction (d2).

10. A microstructure (10) for selective transmission of X-ray or gamma-ray radiation, obtainable by the method according to any one of the preceding claims.

11. The microstructure (10) according to claim 10, wherein the septa wall structures (20) have a wall thickness (w) of less than 200 µm, preferably less than 100 µm, more preferably less than or equal to 70 µm

12. The microstructure (10) according to claim 10 or 11, wherein the septa wall structures (20) have a height (h) to thickness (w) aspect ratio of more than 100, preferably more than 200 and even more preferably more than 400.

13. An imaging component comprising the microstructure (10) of claim 10 or 11 or 12.

14. The imaging component according to claim 13, wherein the imaging component comprises a plurality of stacked microstructures (10).

15. The imaging component according to claim 13 or 14, wherein the imaging component comprises one or more of:
- an X-ray or gamma-ray filter;
- an X-ray or gamma-ray collimator;
- an X-ray or gamma-ray anti-scatter device; and
- an X-ray or gamma-ray grating.
